# EUROPEAN PATENT SPECIFICATION

(11) **EP 3 664 899 B1**
(45) Date of publication and mention of the grant of the patent: **02.12.2026**
(21) Application number: 18768954.2
(22) Date of filing: 12.07.2018
(51) Int. Cl.: A61P 37/00, A23L 33/125, A61K 31/702

(54) **HUMAN MILK OLIGOSACCHARIDE FOR IMPROVING VACCINE SPECIFIC IMMUNE RESPONSE**
MENSCHLICHES MILCHOLIGOSACCHARID ZUR VERBESSERUNG DER IMPFSTOFFSPEZIFISCHEN IMMUNANTWORT
OLIGOSACCHARIDE DE LAIT HUMAIN PERMETTANT L'AMÉLIORATION DE LA RÉPONSE IMMUNITAIRE SPÉCIFIQUE AU VACCIN

(30) Priority: 11.08.2017 WO PCT/NL2017/050532
(43) Date of publication of application: 17.06.2020
(73) Proprietor: N.V. Nutricia, 2712 HM Zoetermeer (NL)
(72) Inventor: POTAPPEL-VAN 'T LAND, Belinda, 3584 CT Utrecht (NL); VAN DEN ELSEN, Lieke Wilhelmina Johanna, 5683 MA Best (NL); STAHL, Bernd, 3584 CT Utrecht (NL)
(74) Representative: Nederlandsch Octrooibureau
(86) International application number: PCT/NL2018/050481
(87) International publication number: WO 2019/031963

(56) References cited:
- EP-A1- 2 662 084
- EP-A1- 2 813 230
- US-A1- 2015 031 645
- US-A1- 2016 316 808
- US-A1- 2016 354 395

## Description

### Field of the invention

The present invention is in the field of infant nutrition involving specific human milk oligosaccharides.

### Background of the invention

Human milk is the preferred food for infants and is also denoted as the golden standard. One of the reasons for this is the relatively high inclusion level of human milk oligosaccharides (HMOs) which are non-digestible oligosaccharides (NDOs). These HMOS form a complex mixture of prebiotic molecules that form an excellent substrate for beneficial microbiota to thrive on. HMOs, being part of mother's milk, are of course mostly relevant to help infants build the beneficial microbiota needed for optimal health.

It is known that the state of the intestinal microbiota is directly linked to one's health. The intestinal microbiota consists of a large association of beneficial bacteria which is believed to be unique for each individual, but which fulfils the same overall physiological functions. These functions include preventing other pathogenic microorganisms from proliferating, ensuring a proper digestive functioning and ensuring a proper intestinal barrier which is important for the immune system to function.

Methods for selectively stimulating the beneficial microbiota include administering live beneficial bacteria (usually denoted as probiotics) to a human subject and administering a substrate for beneficial microbiota to grow on (usually called prebiotics) to a human subject.

The most abundant HMO in human milk is 2'-fucosyllactose (2'-FL). For this oligosaccharide it is known that it can boost the immune system and prevent against infection. For instance, from WO 2015/071391 it is known that 2'-fucosyllactose is beneficial for the prevention and treatment of viral influenza. EP 2 662 084 discloses a nutritional composition comprising 2'-fucosyllactose and betagalacto-oligosaccharides for use in enhancement of vaccination response.

### Summary of the invention

It has now surprisingly been found that the administration of human milk oligosaccharides has an unexpected effect in male subjects on enhancing the vaccination response. More specifically the inventors have found that administering a composition comprising a fucosylated non-digestible human milk oligosaccharide, in particular comprising 2'-fucosyllactose, gives a boost to the vaccination response in male subjects, wherein the male subjects are preferably infants.

This has been experimentally established by vaccinating males and females with a trivalent influenza vaccine and measuring the Ig-G levels after said vaccination. Surprisingly the control group showed that females respond much better to the vaccination than males do. Even more surprising it was shown that it is possible to repair the low vaccination response in the males by administering a composition comprising 2'-fucosyllactose and preferably comprising further non digestible oligosaccharides.

A beneficial effect on enhancing immune response is closely related to the subjects' overall immune fitness. This concept of immune fitness refers to the health of the immune system or the ability of the immune system to neutralize a harmful pathogen. An immune system that is fit has the ability to respond quickly and adequately to a potentially harmful pathogen.

A closely related to immune fitness is immune balance. A healthy immune system is neither overactive nor underactive. Instead it is balanced between these two extremes. Hence a healthy immune system necessarily is a balanced immune system. The invention is defined by the appended set of claims.

### Detailed description of the invention

The present invention thus concerns a composition comprising a fucosylated non-digestible human milk oligosaccharide, for use in enhancing vaccine specific immune response in a human subject through enteral administration, preferably through oral administration, wherein the human subject is an infant male, wherein the fucosylated non-digestible human milk oligosaccharide is 2'-fucosyllactose and wherein the composition further comprises galacto-oligosaccharide and fructo-oligosaccharide, wherein the galacto-oligosaccharide is short chain galacto-oligosaccharide and wherein the fructo-oligosaccharide is long chain fructo-oligosaccharide, wherein the composition comprises 0.15 wt.% to 1 wt.% 2'-fucosyllactose based on dry weight, and wherein the weight ratio of galacto-oligosaccharides and fructo-oligosaccharide is between 20 and 1, wherein the vaccine specific immune response is an IgG response to an influenza vaccine and wherein the composition is administered via an infant formula to said infant, wherein the effect is determined compared to the vaccine specific immune response in a male human subject not being administered the composition comprising 2'-fucosyllactose, short chain galacto-oligosaccharide and long chain fructo-oligosaccharide.

In a preferred embodiment, the influenza vaccine response is in response to a single vaccination. In a further preferred embodiment, the influenza vaccine response is in response to a vaccination with inactivated vaccine, or in other words to a vaccine of inactivated virus particles. Preferably the single vaccination is a single influenza vaccination and more preferably said vaccination is a single trivalent influenza vaccination. Preferably the single vaccination is a single influenza vaccination of inactivated virus particles, and more preferably said vaccination is a single trivalent influenza vaccination of inactivated trivalent virus particles.

In a preferred embodiment, the male infant is in need of having an enhanced vaccine specific immune response. In one embodiment the vaccine specific immune response is a trivalent influenza vaccine response.

### NDOs

Prebiotics are typically indigestible sugar-type compounds such as non-digestible oligosaccharides (NDOs). These compounds pass through the first part of the gastrointestinal tract substantially without being digested. In the intestine these compounds are fermented by the microbiota releasing, amongst others, short chain fatty acids which are adsorbed by the human body.

There are many sources of NDO's, amongst which is human breast milk. Usually these oligosaccharides are denoted as human milk oligosaccharides (HMOs). Typical NDOs used in infant foods are GOS and FOS.

### Human milk oligosaccharides

Human milk is the preferred food for infants and is also denoted as the golden standard. Human milk contains a particularly high level of oligosaccharides of roughly 10 g/L, which is typically much more than the level of NDO in the milk from domestic animals. Also, compared to the NDOs in the milk of domestic animals, HMOs are structurally different. Human NDOs are very complex and consist of a heterogenic group of more than 130 different compounds with a diverse sugar composition. Because of their complex and polymorphic structure, large-scale synthesis is complicated. It is therefore not yet technically and economically feasible to prepare compositions, such as infant formulas, with NDO composition identical to human milk. In the method or use according to the present, a fucosylated non-digestible human milk oligosaccharide is used.

### Fucosylated non-digestible human milk oligosaccharides

Fucosyllactose (FL) is a fucosylated non-digestible oligosaccharide present in human milk. It is not present in bovine milk. It consists of three monosaccharide units, fucose, galactose and glucose linked together. Lactose is a galactose unit linked to a glucose unit via a beta 1,4 linkage. A fucose unit is linked to a galactose unit of a lactose molecule via an alpha 1,2 linkage (2'-fucosyllactose, 2'-FL) or via an alpha 1,3 linkage to the glucose unit of a lactose (3-Fucosyllactose, 3-FL). 2'FL is the most abundant NDO in human milk. The HMO used in the current invention is 2'-FL.

2'-FL, (β-L-Fuc-(1→2)-β-D-Gal-(1→4)-D-Glc) and 3-FL (β-L-Fuc-(1→3)-[β-D-Gal-(1→4)]-D-Glc), are commercially available for instance from Sigma-Aldrich. Alternatively, they can be isolated from human milk, for example as described in Andersson & Donald, 1981, J Chromatogr. 211:170-1744, or produced by genetically modified micro-organisms, for example as described in Albermann et al, 2001, Carbohydrate Res. 334:97-103.

Preferably, a composition according to the invention comprises 1 mg to 3 g 2'-FL per 100 ml, more preferably 10 mg to 2 g, more preferably 20 mg to 200 mg, even more preferably 20 mg to 150 mg 2'-FL per 100 ml. Based on dry weight, the present composition comprises 0.15 wt.% to 1 wt.% 2'-FL. A lower amount of fucosyllactose will be less effective in stimulating the immune system, whereas a too high amount will result in unnecessary high costs of the product.

### Non-digestible oligosaccharides other than human milk oligosaccharides

The nutritional composition preferably comprises non-digestible oligosaccharides other than HMO. Preferably the NDO other than HMO stimulates the growth of bifidobacteria and/or lactobacilli, more preferably bifidobacteria. An increased content of bifidobacteria and/or lactobacilli stimulate the formation of a healthy intestinal microbiota. The NDO are preferably not or only partially digested in the intestine by the action of acids or digestive enzymes present in the human upper digestive tract, in particular in the small intestine and stomach, and are fermented by the human intestinal microbiota. For example, sucrose, lactose, maltose and the common maltodextrins are considered digestible.

Preferably the present composition comprises non-digestible oligosaccharides with a DP in the range of 2 to 250, more preferably 2 to 60.

The composition comprises long chain fructo oligosaccharides and short chain galacto-oligosaccharides.

The galacto-oligosaccharides preferably are beta-galacto-oligosaccharides. Beta-galacto-oligosaccharides are also referred to as trans-galacto-oligosaccharides (TOS). Beta-galacto-oligosaccharides are for example sold under the trademark Vivinal(TM) (Borculo Domo Ingredients, Netherlands). Another suitable source is Bi2Munno (Clasado). Preferably the TOS comprises at least 80 % beta-1,4 and beta-1,6 linkages based on total linkages, more preferably at least 90 %.

Fructo-oligosaccharide is a NDO comprising a chain of beta-linked fructose units with a DP or average DP of 2 to 250, more preferably 2 to 100, even more preferably 10 to 60. Fructo-oligosaccharide includes inulin, levan and/or a mixed type of polyfructan. An especially preferred fructo-oligosaccharide is inulin. Fructo-oligosaccharide suitable for use in the compositions is also commercially available, e.g. Raftiline^{®}HP (Orafti). Preferably the fructo-oligosaccharide has an average DP above 20.

In a preferred embodiment the composition comprises a mixture of inulin and short chain fructo-oligosaccharides. A mixture of at least two different non-digestible oligosaccharides advantageously stimulates the beneficial bacteria of the intestinal microbiota to a greater extent. The weight ratio in a mixture of galacto-oligosaccharides and fructo-oligosaccharide, is between 20 and 1. Galacto-oligosaccharides, preferably beta-galacto-oligosaccharides, are more capable of stimulating bifidobacteria. Preferably the present composition comprises galacto-oligosaccharides, preferably beta-galacto-oligosaccharides, with a degree of polymerization (DP) of 2 to 10 and/or fructo-oligosaccharides with a DP of 2 to 60.

In a preferred embodiment the composition does not comprise non-digestible acid oligosaccharides other than HMO. In particular, in a preferred embodiment the composition does not comprise non-digestible acid oligosaccharides that are derived from pectin. Such non-digestible acid oligosaccharides are characterized by the presence of uronic acid containing residues. Thus, in a preferred embodiment the composition does not comprise non-digestible uronic acid oligosaccharides, preferably the composition does not comprise non-digestible uronic acid oligosaccharides with a degree of polymerization of 2 to 250. The term non-digestible uronic acid oligosaccharide as used herein refers to a non-digestible oligosaccharide wherein at least 50% of the residues are selected from the group consisting of guluronic acid, mannuronic acid, galacturonic acid and glucuronic acid.

### Compositions

The present invention advantageously concerns a composition wherein the lipid provides 5 to 50% of the total calories, the protein provides 5 to 50% of the total calories, and the carbohydrate provides 15 to 90% of the total calories. Preferably, in the present composition the lipid provides 35 to 50% of the total calories, the protein provides 7.5 to 12.5% of the total calories, and the carbohydrate provides 40 to 55% of the total calories. For calculation of the % of total calories for the protein component, the total of energy provided by the proteins, peptides and amino acids needs to be taken into account.

The present composition preferably comprises at least one lipid selected from the group consisting of animal lipid (excluding human lipids) and vegetable lipids. Preferably the present composition comprises a combination of vegetable lipids and at least one oil selected from the group consisting of fish oil, animal oil, algae oil, fungal oil, and bacterial oil. The present composition comprising non-digestible oligosaccharides and B. breve excludes human milk.

The present composition preferably comprises protein. The protein component used in the nutritional preparation are preferably selected from the group consisting of non-human animal proteins (preferably milk proteins, preferably proteins from cow's milk), vegetable proteins (preferably soy protein and/or rice protein), free amino acids and mixtures thereof. The present composition preferably contains casein, whey, hydrolysed casein and/or hydrolysed whey protein. Preferably the protein comprises intact proteins, more preferably intact bovine whey proteins and/or intact bovine casein proteins.

The present composition preferably comprises digestible carbohydrates. The present composition preferably comprises a digestible carbohydrate component, wherein at least 35 wt.%, more preferably at least 50 wt.%, more preferably at least 75 wt.%, even more preferably at least 90 wt.%, most preferably at least 95 wt.% is lactose. The present composition preferably comprises at least 25 grams lactose per 100 gram dry weight of the present composition, preferably at least 40 grams lactose/100 gram.

The liquid nutritional composition preferably has a caloric density between 0.1 and 2.5 kcal/ml, even more preferably a caloric density of between 0.5 and 1.5 kcal/ml, most preferably between 0.6 and 0.8 kcal/ml. The amount of nutritional composition administered per day is preferably between 50 and 2000 ml, more preferably between 200 and 1500, most preferably between 400 and 1000 ml.

In a preferred embodiment the preterm formula comprises from 5 to 25 wt.% protein, preferably 9 to 20 wt.%, more preferably 13 to 18 wt.% protein based on the dry weight of the preterm formula. In a preferred embodiment the preterm formula comprises from 1.8 to 3.0 g protein, preferably, preferably 2.0 to 3.0 g, preferably 2.5 g to 2.6 g protein, per 100 ml.

In one embodiment the present invention concerns a supplement, suitable to fortify human milk, to fortify human milk fortified with a standard human milk fortifier or to fortify a standard preterm formula. In the context of this invention, a supplement does not comprise all macro- and micronutrients needed for preterm infants so as to achieve a growth similar to fetal growth coupled with satisfactory functional development.

Thus in one embodiment the nutritional composition for use according to the present invention comprises protein, fat and/or digestible carbohydrates and is selected from the group consisting of an infant starter formula, an infant follow on formula, a toddler milk, a preterm formula, a post discharge formula and a human milk fortifier.

### Application

The present use is specifically intended for infants. Infants have an age of 0-12 months, toddlers have an age of 12-36 months.

The present composition is enterally administered, more preferably orally. The present composition is preferably a nutritional formula, preferably an infant formula. The present composition can advantageously be applied as a complete nutrition for infants. The present composition preferably comprises lipid, protein, and carbohydrate and is preferably administered in liquid form. The present invention includes dry compositions, e.g. powders, which are accompanied with instructions as to admix said dry compositions, in particular nutritional formula, with a suitable liquid, e.g. water.

The present invention of enhancing vaccine specific immune response in a male human subject comprises administering a composition comprising the non-digestible human milk oligosaccharide according to the invention, to the male infant and the composition is administered via an infant formula. Benefits of this mode of administration include minimizing additional handlings for the caretaker, certainty about the administered dose as well as certainty of administration.

In one embodiment, the composition is administered via the mother during pregnancy and/or breast feeding. This comprises supplementing the diet of the mother with a composition comprising the fucosylated non-digestible human milk oligosaccharide, combined with galacto-oligosaccharide and fructo-oligosaccharide. The benefit of administering the present composition via the mother during pregnancy and/or breast feeding is that this enhances the vaccination response at the earliest age possible. After breast feedings has stopped, the composition is preferably administered via the infant formula as this ensures continuous administration of the composition and an optimally enhanced vaccination response.

In one embodiment the administration of the composition according to the invention starts prior to vaccination. Hence, preferably the composition according to the invention is administered during pregnancy and/or infancy, which is from 0-12 months, and the enhanced vaccine specific immune response is later-in-life, preferably in adulthood.

Vaccination is a method for protection against specific diseases. Usually the method of vaccination involves administering a composition called a vaccine comprising an antigen to a subject which results in a boost of the immune system. There are multiple types of vaccines. Firstly there is the inactivated vaccine which (largely) comprises dead pathogens. Secondly there is the attenuated vaccine which comprises weakened pathogens. Then there are virus-like particles that resemble viruses but are much less pathogenic. And finally there is the subunit vaccine which comprises the antigen but not the viral particles. In one embodiment, the present invention of enhancing vaccine specific immune response is a vaccine response following vaccination with inactivated vaccine. In one embodiment, the present invention of enhancing vaccine specific immune response is a vaccine response following a single vaccination, preferably following a single vaccination with inactivated vaccine.

The enhanced vaccine specific immune response is an enhanced IgG response.

The effectiveness of vaccination depends on a large number of variables, which can be split-up in vaccine-related vaccination effects and subject-related vaccination effects.

The vaccine-related vaccination effects comprise the effectiveness of the particular vaccine, its concentration and how often it is administered.

The subject-related vaccination effects include the subjects general health but also its susceptibility for vaccination. The subject's general health effect resides not only in that healthy humans are less likely to become ill, but also in that they have better chances of a quick recover. These effects are closely related to the subjects overall immune fitness. This concept of immune fitness refers to the health of the immune system or the ability of the immune system to neutralize a harmful pathogen.

An immune system that is fit has the ability to respond quickly and adequately to a potentially harmful pathogen.

A closely related concept is the immune balance. The immune system needs to be respond adequately and quickly to any protruding pathogen. When the immune system reacts too late or when the reaction is not adequate, the immune system is said to be underactive. Subjects with an underactive immune system have an increased susceptibility to infections and diseases. On the other hand when the immune system can be overactive. This also leads to physical complaints such as autoimmune diseases or allergies. A good example of an overactive immune system is pollen allergy where harmless pollen are mistakenly seen as pathogens. The immune system mistakenly reacts to the harmless pollen causing all sorts of complaints. A healthy immune system is neither overactive nor underactive. Instead it is balanced between these two extremes. Hence a healthy immune system necessarily is a balanced immune system.

A widely-used vaccine is the influenza vaccine, which helps protecting against the influenza virus. Influenza vaccines are available in different forms. There are, for instance, the trivalent or quadrivalent injections which contain the inactivated form of the virus. Also there is the nasal spray which comprises the attenuated form of the virus. In a preferred embodiment, the enhanced influenza vaccination response in males of the invention is an enhanced influenza vaccination response to a vaccination with inactivated virus.

In a preferred embodiment, the enhanced influenza vaccination response in males of the invention is an enhanced influenza vaccination response to a single vaccination, preferably a single trivalent influenza vaccination. In a preferred embodiment, the enhanced influenza vaccination response in males of the invention is an enhanced influenza vaccination response to a single vaccination with inactivated virus, preferably a single trivalent influenza vaccination of inactivated virus particles.

As the influenza virus rapidly changes, new vaccines are produced often. This is usually done in fertilized chicken eggs. To produce the trivalent vaccine, three strains (H1N1, H3N2, and a B-strain) are isolated and subsequently used to make the vaccine.

### Examples

### Example 1: Immune response of male and female mice on a prebiotic diet

### Materials and Methods

BALB/c breeding pairs were fed either control diet (AIN93G) from the day of timed mating or a prebiotic diet containing short-chain galacto-oligosaccharides, long-chain fructo-oligosaccharides and the human milk specific oligosaccharide 2'-FL (scGOS at 0.9 wt.% / IcFOS at 0.1 wt.% / 2'-FL at 1 wt.% (total of 2 wt.%)). The AIN93G diet in the control group was adjusted with cellulose fibre to provide comparable diets and fully compensated for the presence of carrier material used to introduce the Vivinal^{®} GOS syrup (scGOS), Inulin HP^{®} (IcFOS fibre) and 2'-FL. 1/3rd of the litters from the control breeding pairs were switched to prebiotic diet within 24 hours after birth or at weaning and maintained on this throughout the course of the experiment. At 6 weeks of age male and female offspring of all 4 dietary groups were immunized subcutaneously in the scruff of the neck with 1:5 of the human adult dose of trivalent influenza vaccine (TIV, Fluvax 2015) per mouse (100 µl vaccine + 100 µl PBS). Mice were tail bled at days 0, 28 and 49, serum was stored at -20°C until analysis for TIV-specific antibodies by ELISA. Optical density measurements were used to quantify the ELISA colour change.

### Results

Only in males receiving the diet comprising 2'-FL, a significant higher concentration of IgG2a, a statistically significant (p <0.01) higher concentration of IgG1 and a statistically significant (p <0.05) higher concentration of total IgG compared to the control group was found. This finding shows that the males have a significant increase in vaccine specific antibody response due to this fucosylated HMO, hence have an enhanced vaccine specific immune response. This result is indicative for an improved immune fitness and/or restoration of immune balance.

### Example 2: Infant formula for improving immune fitness, restoration of immune balance and/or enhancing vaccine specific immune response

An infant formula according to the invention comprising per 100 ml (13.9 dry weight):
1.4 g protein (whey and casein)
7.3 g digestible carbohydrates (including lactose)
3.6 g fat (vegetable fat, fish oil)
0.6 g non-digestible oligosaccharides of which 60 mg 2'-fucosyllactose and 480 mg beta-galacto-oligosaccharides, and 60 mg fructo-oligosaccharides

Further are included: choline, myo-inositol, taurine, minerals, trace elements, and vitamins in accordance with guidelines.

## Claims

1. A composition comprising a fucosylated non-digestible human milk oligosaccharide, for use in enhancing vaccine specific immune response in a human subject through enteral administration, preferably through oral administration, wherein the human subject is an infant male, wherein the fucosylated non-digestible human milk oligosaccharide is 2'-fucosyllactose and wherein the composition further comprises galacto-oligosaccharide and fructo-oligosaccharide, wherein the galacto-oligosaccharide is short chain galacto-oligosaccharide and wherein the fructo-oligosaccharide is long chain fructo-oligosaccharide, wherein the composition comprises 0.15 wt.% to 1 wt.% 2'-fucosyllactose based on dry weight, and wherein the weight ratio of galacto-oligosaccharides and fructo-oligosaccharide is between 20 and 1, wherein the vaccine specific immune response is an IgG response to an influenza vaccine and wherein the composition is administered via an infant formula to said infant, wherein the effect is determined compared to the vaccine specific immune response in a male human subject not being administered the composition comprising 2'-fucosyllactose, short chain galacto-oligosaccharide and long chain fructo-oligosaccharide.

2. The composition comprising a fucosylated non-digestible human milk oligosaccharide for use according claim 1 wherein the composition comprising a fucosylated non digestible human milk oligosaccharide does not comprise non-digestible uronic acid oligosaccharides.

3. The composition comprising a fucosylated non-digestible human milk oligosaccharide for use according to any of the preceding claims wherein the vaccine specific immune response is a trivalent influenza vaccine response.

4. The composition comprising a fucosylated non-digestible human milk oligosaccharide for use according to any of the preceding claims wherein the vaccine specific immune response is a vaccine response after vaccination with inactivated vaccine.

5. The composition comprising a fucosylated non-digestible human milk oligosaccharide for use according to any of the preceding claims wherein the vaccine specific immune response is a vaccine response after single vaccination.

6. The composition comprising a fucosylated non-digestible human milk oligosaccharide for use according to any of the preceding claims wherein the vaccine specific immune response is a vaccine response after a single vaccination with inactivated vaccine.

## Patentansprüche

1. Zusammensetzung, umfassend ein fucosyliertes nicht-verdauliches humanes Milch-Oligosaccharid, zur Verwendung zum Verstärken der impfstoffspezifischen Immunantwort bei einer menschlichen Person durch enterale Verabreichung, vorzugsweise durch orale Verabreichung, wobei die menschliche Person ein männlicher Säugling ist, wobei das fucosylierte nicht-verdauliche humane Milch-Oligosaccharid 2'-Fucosyllactose ist und wobei die Zusammensetzung ferner Galacto-Oligosaccharid und Fructo-Oligosaccharid umfasst, wobei das Galacto-Oligosaccharid kurzkettiges Galacto-Oligosaccharid ist und wobei das Fructo-Oligosaccharid langkettiges Fructo-Oligosaccharid ist, wobei die Zusammensetzung 0,15 Gew.-% bis 1 Gew.-% 2'-Fucosyllactose bezogen auf das Trockengewicht umfasst, und wobei das Gewichtsverhältnis von Galacto-Oligosacchariden und Fructo-Oligosaccharid zwischen 20 und 1 liegt, wobei die impfstoffspezifische Immunantwort eine IgG-Antwort auf einen Influenza-Impfstoff ist und wobei die Zusammensetzung dem Säugling über eine Säuglingsnahrung verabreicht wird, wobei der Effekt im Vergleich zur impfstoffspezifischen Immunantwort bei einer männlichen menschlichen Person, der die Zusammensetzung, umfassend 2'-Fucosyllactose, kurzkettiges Galacto-Oligosaccharid und langkettiges Fructo-Oligosaccharid, nicht verabreicht wird, bestimmt wird.

2. Zusammensetzung, umfassend ein fucosyliertes nicht-verdauliches humanes Milch-Oligosaccharid, zur Verwendung nach Anspruch 1, wobei die Zusammensetzung, umfassend ein fucosyliertes nicht-verdauliches humanes Milch-Oligosaccharid, keine nicht-verdaulichen Uronsäure-Oligosaccharide umfasst.

3. Zusammensetzung, umfassend ein fucosyliertes nicht-verdauliches humanes Milch-Oligosaccharid, zur Verwendung nach einem der vorhergehenden Ansprüche, wobei die impfstoffspezifische Immunantwort eine Impfstoffantwort auf einen trivalenten Influenza-Impfstoff ist.

4. Zusammensetzung, umfassend ein fucosyliertes nicht-verdauliches humanes Milch-Oligosaccharid, zur Verwendung nach einem der vorhergehenden Ansprüche, wobei die impfstoffspezifische Immunantwort eine Impfstoffantwort nach einer Impfung mit einem inaktivierten Impfstoff ist.

5. Zusammensetzung, umfassend ein fucosyliertes nicht-verdauliches humanes Milch-Oligosaccharid, zur Verwendung nach einem der vorhergehenden Ansprüche, wobei die impfstoffspezifische Immunantwort eine Impfstoffantwort nach einmaliger Impfung ist.

6. Zusammensetzung, umfassend ein fucosyliertes nicht-verdauliches humanes Milch-Oligosaccharid, zur Verwendung nach einem der vorhergehenden Ansprüche, wobei die impfstoffspezifische Immunantwort eine Impfstoffantwort nach einer einmaligen Impfung mit einem inaktivierten Impfstoff ist.

## Revendications

1. Composition comprenant un oligosaccharide fucosylé non digestible du lait maternel humain, pour une utilisation dans l'amélioration de la réponse immunitaire spécifique au vaccin chez un sujet humain par administration entérale, de préférence par administration orale, dans laquelle le sujet humain est un nourrisson de sexe masculin, dans laquelle l'oligosaccharide fucosylé non digestible du lait maternel humain est le 2'-fucosyllactose et dans laquelle la composition comprend en outre du galacto-oligosaccharide et du fructo-oligosaccharide, dans laquelle le galacto-oligosaccharide est du galacto-oligosaccharide à chaîne courte et dans laquelle le fructo-oligosaccharide est du fructo-oligosaccharide à longue chaîne, dans laquelle la composition comprend de 0,15 % en poids à 1 % en poids de 2'-fucosyllactose sur la base du poids sec, et dans laquelle le rapport en poids des galacto-oligosaccharides et du fructo-oligosaccharide est entre 20 et 1, dans laquelle la réponse immunitaire spécifique au vaccin est une réponse IgG à un vaccin antigrippal et dans laquelle la composition est administrée via une préparation pour nourrissons audit nourrisson, dans laquelle l'effet est déterminé par comparaison avec la réponse immunitaire spécifique au vaccin chez un sujet humain de sexe masculin ne recevant pas la composition comprenant du 2'-fucosyllactose, du galacto-oligosaccharide à chaîne courte et du fructo-oligosaccharide à longue chaîne.

2. Composition comprenant un oligosaccharide fucosylé non digestible du lait maternel humain pour une utilisation selon la revendication 1 dans laquelle la composition comprenant un oligosaccharide fucosylé non digestible du lait maternel humain ne comprend pas d'oligosaccharides non digestibles d'acide uronique.

3. Composition comprenant un oligosaccharide fucosylé non digestible du lait maternel humain pour une utilisation selon l'une quelconque des revendications précédentes dans laquelle la réponse immunitaire spécifique au vaccin est une réponse au vaccin antigrippal trivalent.

4. Composition comprenant un oligosaccharide fucosylé non digestible du lait maternel humain pour une utilisation selon l'une quelconque des revendications précédentes dans laquelle la réponse immunitaire spécifique au vaccin est une réponse vaccinale après vaccination avec un vaccin inactivé.

5. Composition comprenant un oligosaccharide fucosylé non digestible du lait maternel humain pour une utilisation selon l'une quelconque des revendications précédentes dans laquelle la réponse immunitaire spécifique au vaccin est une réponse vaccinale après vaccination unique.

6. Composition comprenant un oligosaccharide fucosylé non digestible du lait maternel humain pour une utilisation selon l'une quelconque des revendications précédentes dans laquelle la réponse immunitaire spécifique au vaccin est une réponse vaccinale après une vaccination unique avec un vaccin inactivé.
